# EUROPEAN PATENT APPLICATION

(11) **EP 4 205 713 A1**
(43) Date of publication of application: **05.07.2023**
(21) Application number: 21861315.6
(22) Date of filing: 17.08.2021
(51) Int. Cl.: A61F 13/15, A61F 13/476, A61F 13/511, A61F 13/56

(54) **INDIVIDUALLY PACKAGED ABSORBENT ARTICLE**

(30) Priority: 25.08.2020 JP 2020141414
(71) Applicant: DAIO PAPER CORPORATION, Shikokuchuo-shi Ehime 799-0492 (JP)
(72) Inventor: KURAMOCHI, Keisuke, Sakura-shi, Tochigi 329-1411 (JP)
(74) Representative: Epping - Hermann - Fischer
(86) International application number: PCT/JP2021/030043
(87) International publication number: WO 2022/044887

(57) **Abstract**

Provided is an individually-wrapped sanitary napkin N, in which a sanitary napkin 1 is folded lengthwise with a wrapping sheet 10 and wrapped individually, the sanitary napkin 1 including an inner surface sheet 3 that constitutes a skin-contacting surface layer and a pair of wing-like flaps W that have wing adhesive layers 9 for fixing the wing-like flaps W to the underwear. The wing-like flaps W are both folded to the skin-contacting surface side, and the wing adhesive layers 9, provided in the folded portions of the wing-like flaps W, are collectively covered by a wing separating sheet 12, and a functional chemical 20 having a specific function is contained in the inner surface sheet 3 in the area where the wing separating sheet 12 is layered.

## Description

### TECHNICAL FIELD

The present invention relates to an individually-wrapped absorbent article, in which an absorbent article such as a sanitary napkin, a panty liner, an incontinence pad, or the like is wrapped individually in a wrapping sheet. More specifically, the present invention relates to an individually-wrapped absorbent article, in which an absorbent article, containing a functional chemical that has a specific function in a predetermined area of an inner surface sheet, is wrapped individually.

### BACKGROUND ART

Conventionally, absorbent articles that contain a functional chemical such as a skin care agent, a deodorant, a fragrance, a cooling sensitizer, and the like have been known. For example, Patent Document 1 below discloses an absorbent article that is configured such that a fragrant area is covered by a flap sheet, where this flap sheet serves as a separating sheet, and where a first longitudinal fold and a second longitudinal fold serve as base points.

Furthermore, Patent Document 2 below discloses an absorbent article, in which a functional material area containing a functional material is provided between an absorbent layer and an inner surface sheet, and a compressed portion is formed by compressing together the functional material area and an adjacent layer that lies adjacent to the functional material area in the absorbent article's thickness direction.

### RELATED-ART DOCUMENTS

### PATENT DOCUMENTS

[Patent Document 1] Japanese Unexamined Patent Application Publication No. 2013-208318
[Patent Document 2] Japanese Unexamined Patent Application Publication No. 2016-67749

### SUMMARY OF THE INVENTION

### PROBLEM TO BE SOLVED BY THE INVENTION

Above Patent Document 1 describes that covering the fragrant area with a flap sheet that serves as a separating sheet brings about an advantage of minimizing the dissipation of the fragrance ingredient prior to use. However, since the separating sheet covers only a part of the functional chemical, when the functional chemical is contained in an inner surface sheet, there is a risk that the functional chemical transfers to the portion of the absorbent article that is folded and layered on top of the functional chemical, and soils the clothes when the absorbent article is worn.

Furthermore, according to Patent Document 2, although a functional area is provided between an absorbent layer and an inner surface sheet, no sheet for covering this functional area is provided. Consequently, there is a risk that the fragrance ingredient such as a perfume volatilizes prior to use, and its effect will be significantly reduced at the time of use.

The present invention has been made in view of the foregoing and is primarily intended to provide an individually-wrapped absorbent article that can prevent a functional chemical from transferring to an unintended portion, and that can also prevent the functional chemical from dissipating prior to use, so that the functional chemical can exert its effect optimally when in use.

### MEANS TO SOLVE THE PROBLEM

To solve the above problems, a first aspect of the present invention provides an individually-wrapped absorbent article, in which an absorbent article is folded lengthwise with a wrapping sheet and wrapped individually. The absorbent article has an inner surface sheet that constitutes a skin-contacting surface layer and a pair of wing-like flaps that have wing adhesive layers for fixing the wing-like flaps to the underwear. Both wing-like flaps are folded to the skin-contacting surface side, and the wing adhesive layers, provided in the folded portions of the wing-like flaps, are collectively covered by a separating sheet, and a functional chemical having a specific function is contained in the inner surface sheet in the area where the separating sheet is layered.

According to this first aspect of the present invention, the functional chemical is contained in the inner surface sheet in the area where the separating sheet for covering the wing adhesive layers of the wing-like flaps is layered, so that, when the absorbent article is folded lengthwise with the wrapping sheet and wrapped individually, the functional chemical is prevented from transferring to an unintended portion, and therefore there is no risk of soiling the clothes of the person wearing the absorbent article. In addition, since the functional chemical contained in the inner surface sheet is covered by the separating sheet in an individually-wrapped state, it is possible to prevent the functional chemical from dissipating prior to use, thereby allowing the functional chemical to exert its function optimally when in use.

A second aspect of the present invention provides the individually-wrapped absorbent article of claim 1, in which the functional chemical is provided in at least one of the following manners: the functional chemical is provided over the entire surface of a predetermined area; a plurality of functional chemicals are provided at intervals in the longitudinal direction or the width direction of the absorbent article; and the plurality of functional chemicals are provided at intervals in the longitudinal direction and the width direction of the absorbent article.

The second aspect of the present invention sets forth the manner in which the functional chemical may be provided. As long as within the area where the separating sheet is layered, the functional chemical may be provided over the entire surface of this area. Alternatively, multiple functional chemicals may be provided at intervals in the longitudinal direction or the widthwise direction of the absorbent article, or provided at intervals in the longitudinal direction and the widthwise direction.

A third aspect of the present invention provides the individually-wrapped absorbent article of one of claims 1 and 2, in which the wing-like flaps are folded in both side portions that do not directly contact the inner surface sheet.

According to the third aspect of the present invention, when the absorbent article is wrapped individually, the wing-like flaps are folded in both side portions that do not directly contact the inner surface sheet, because if the wing-like flaps are folded so as to come in direct contact with the inner surface sheet where the functional chemical is contained, the functional chemical might transfer to the wing-like flaps and soil the clothes of the person wearing the absorbent article.

A fourth aspect of the present invention provides the individually-wrapped absorbent article of claim 3, in which the wing-like flaps are folded with the separating sheet at fold line positions where the wing-like flaps are folded back along the crotch portion of the underwear when the absorbent article is worn.

The fourth aspect of the present invention provides a first means for preventing creases from being made when the wing-like flaps are folded in both side portions that do not directly contact the inner surface sheet so as not to let the functional chemical transfer to the wing-like flaps. These creases are made in positions that are different from the originally intended fold lines for folding the wing-like flaps when wearing the absorbent article by wrapping the wing-like flaps around the crotch portion of underwear, and therefore make it difficult to wear the absorbent article. By means of this first means, the wing-like flaps are folded with the separating sheet at fold line positions where the wing-like flaps are folded back along the crotch portion of underwear when wearing the absorbent article, so as to make the creases at the fold lines for when wearing the absorbent article.

A fifth aspect of the present invention provides the individually-wrapped absorbent article of claim 5, in which fold assist lines are provided at the fold line positions where the wing-like flaps are folded back along the crotch portion of the underwear when the absorbent article is worn.

The fifth aspect of the present invention provides a second means for preventing creases from being made when the wing-like flaps are folded in both side portions that do not directly contact the inner surface sheet so as not to let the functional chemical transfer to the wing-like flaps. These creases are made in positions that are different from the originally intended fold lines for folding the wing-like flaps when wearing the absorbent article by wrapping the wing-like flaps around the crotch portion of underwear, and therefore make it difficult to wear the absorbent article. By means of this second means, fold assist lines that are created by using heat sealing or by using embossed portions are provided at fold line positions where the wing-like flaps are folded back along the crotch portion of underwear when wearing the absorbent article, so as to make the creases at the fold lines for when wearing the absorbent article.

A sixth aspect of the present invention provides the individually-wrapped absorbent article of any one of claims 1 to 5, in which side non-woven fabrics are provided lengthwise in both side portions of the skin-contacting surface side, and the functional chemical is contained in the inner surface sheet between the side non-woven fabrics of both side portions.

According to the sixth aspect of the present invention, when the side non-woven fabrics are provided lengthwise in both side portions on the skin-contacting surface, the functional chemical is contained in the inner surface sheet between the side non-woven fabrics of both side portions. Therefore, the functional chemical is securely contained in the portion constituting the skin-contacting surface of the inner surface sheet, and the effect of the functional chemical acts more readily on the skin of the person wearing the absorbent article.

### EFFECTS OF THE INVENTION

As described in detail above, according to the present invention, it is possible to prevent a functional chemical from transferring to an unintended portion, and prevent the functional chemical from dissipating prior to use, so that the functional chemical can exert its function optimally when in use.

### BRIEF DESCRIPTION OF THE DRAWINGS

[FIG.1] FIG. 1 is a perspective view illustrating an individually-wrapped sanitary napkin N according to the present invention;
[FIG.2] FIG. 2 is a partially broken exploded view illustrating a sanitary napkin 1;
[FIG.3] FIG. 3 is a view taken along line III-III in FIG. 2;
[FIG.4] FIG. 4 is a plan view illustrating a summary of how to wrap the sanitary napkin 1 individually (pattern 1);
[FIG.5] FIG. 5 is a plan view illustrating a summary of how to wrap the sanitary napkin 1 individually (pattern 2);
[FIG. 6] FIG. 6 is a view taken along line VI-VI in FIG. 5;
[FIG. 7] FIGs. 7 are enlarged plan views of main parts of a sanitary napkin 1 according to a modification;
[FIG. 8] FIGs. 8 are enlarged plan views of main parts of the sanitary napkin 1 according to the modification;
[FIG. 9] FIG. 9 is a plan view illustrating a summary of how to wrap the sanitary napkin 1 according to the modification individually (pattern 1);
[FIG.10] FIG. 10 is a plan view illustrating a summary of how to wrap the sanitary napkin 1 according to the modification individually (pattern 2);
[FIG.11] FIG. 11 is a plan view of the sanitary napkin 1, illustrating a first means for assisting the positions to fold wing-like flaps W when wearing the sanitary napkin 1;
[FIG.12] FIG. 12 is a plan view of the sanitary napkin 1 showing a second means for assisting the positions to fold the wing-like flaps W when wearing the sanitary napkin 1; and
[FIG.13] FIG. 13 is a view taken along line XIII-XIII in FIG. 12.

### MODE FOR CARRYING OUT THE INVENTION

Hereinafter, embodiments of the present invention will be described in detail with reference to the accompanying drawings.

In the individually-wrapped absorbent article according to the embodiments of the present invention, an absorbent article such as a sanitary napkin, a panty liner, an incontinence pad, or the like is wrapped individually with a wrapping sheet. To illustrate one embodiment of the absorbent article, a sanitary napkin will be described below as an example.

[Basic structure of individually-wrapped sanitary napkin N]

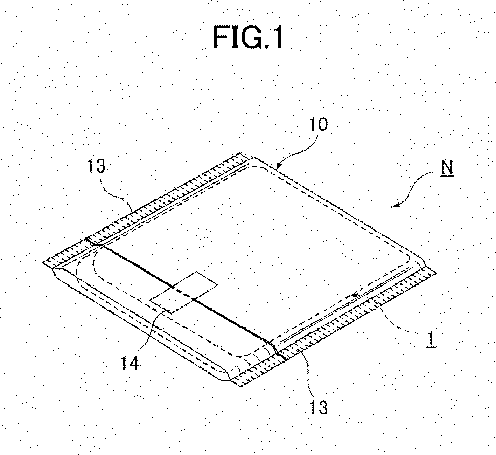

As illustrated in FIG. 1, an individually-wrapped sanitary napkin N according to the present invention is composed of a sanitary napkin 1 and a wrapping sheet 10 for wrapping the sanitary napkin 1 individually. Note that the reference numeral 14 is an adhesive fastening tape that is provided to aid the sealing of the open end portion, and can be omitted.

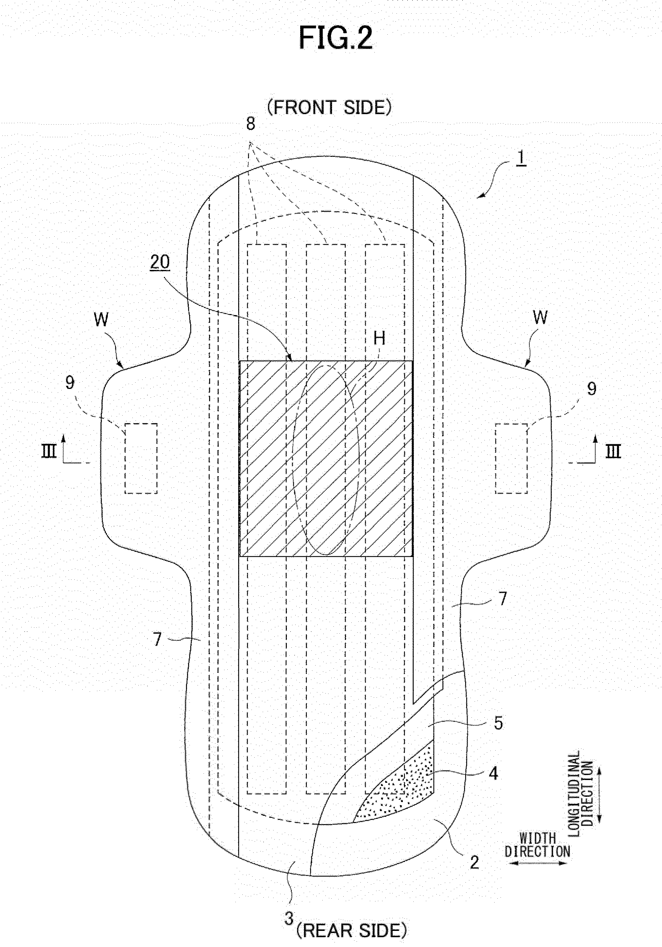

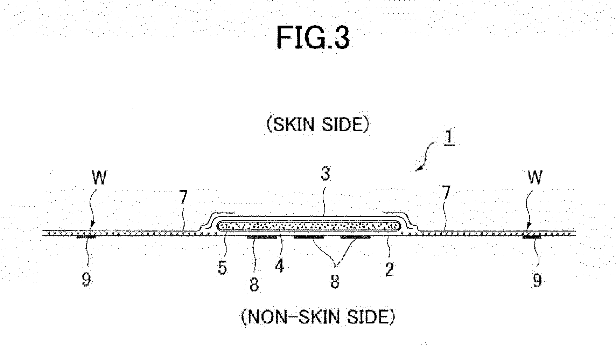

As illustrated in FIG. 2 and FIG. 3, the sanitary napkin 1 includes a liquid-impermeable outer surface sheet 2 made of a polyethylene sheet and the like, a liquid-permeable inner surface sheet 3 that allows menstrual blood, vaginal discharge, and the like (hereinafter also collectively referred to as "body fluids") to permeate fast, an absorber 4 that is made of a cotton-like pulp, a synthetic pulp, or the like, and interposed between these two sheets 2 and 3, and side non-woven fabrics 7 that are provided lengthwise in both side portions of the skin-contacting surface side, substantially over the entire length. Furthermore, looking at the vicinity of the absorber 4, in its front and rear edge portions, outer edge portions of the outer surface sheet 2 and the inner surface sheet 3 are joined by an adhesive means such as hot melting or a joint means such as heat sealing, ultrasonic sealing, and the like. Furthermore, in both side edge portions of this, the side non-woven fabrics 7 and the outer surface sheet 2 extending sideways beyond the absorber 4 are joined by an adhesive means such as hot melting or a joint means such as heat sealing, ultrasonic sealing, and the like. By this means, flap portions, in which there is no intervening absorber, are formed. Note that, in the example illustrated, the absorber 4 is surrounded by a wrapping sheet 5 made of crepe paper, a non-woven fabric, or the like, in order to maintain the shape of the absorber 4 and for improved diffusivity, but this wrapping sheet 5 need not be provided. Furthermore, although not illustrated, a second sheet made of a hydrophilic non-woven fabric or the like and having substantially the same shape as the inner surface sheet 3 may be provided adjacent to the inner surface sheet 3 on the non-skin side.

Below, the structure of the sanitary napkin 1 will be described in more detail. Although a sheet material that is at least water-impervious such as polyethylene is used for the outer surface sheet 2, it is desirable to use a moisture-permeable sheet material from the viewpoint of preventing stuffiness. For this water-impervious/moisture-permeable sheet material, a microporous sheet that may be obtained by forming a sheet by melting and kneading an inorganic filler in an olefin resin such as polyethylene or polypropylene, and then stretching this sheet uniaxially or biaxially may be suitable for use. For the outer surface sheet 2, a polyethylene-laminated non-woven fabric, in which a plastic film and a non-woven fabric are layered, may be used.

Next, for the inner surface sheet 3, a perforated or non-perforated non-woven fabric, a porous plastic sheet, and the like are suitable for use. As for the material fiber to constitute the non-woven fabric, for example, synthetic fiber such as olefins including polyethylene and polypropylene, polyesters, polyamides, and the like, recycled fiber such as rayon and cupra, and natural fiber such as cotton can be used. Non-woven fabrics produced by appropriate processing methods such as spun lacing method, spun bonding method, thermal bonding method, melt blown method, needle punching method, and the like can be used as well. Of these processing methods, the spun lacing method is preferable in that it can produce excellent flexibility, the spun bonding method is preferable in that it can produce excellent drape, and the thermal bonding method is preferable in that it can produce luxuriousness and excellent compressive resilience. When a large number of through holes are formed in the inner surface sheet 3, body fluids are absorbed quickly, which gives an excellent dry touch. The fiber of the non-woven fabric may be either long fiber or short fiber, but short fiber is more preferable for use because it can produce the texture of terrycloth. Also, to facilitate embossing, it is preferable to use olefin fiber such as polyethylene or polypropylene having a relatively low melting point. Furthermore, composite fiber of core-sheath fiber formed with fiber having a high melting point as the core and fiber having a low melting point as the sheath, side-by-side fiber, split fiber, and the like are also suitable for use.

The absorber 4 that is interposed between the outer surface sheet 2 and the inner surface sheet 3 is composed of, for example, a cotton-like pulp and a water-absorbent polymer. The water-absorbent polymer is mixed, in the form of a granular powder, for example, in the pulp constituting the absorber. Examples of the pulp include those made of cellulose fiber and artificial cellulose fiber such as rayon and acetate, including chemical pulp obtained from wood, dissolved pulp, and the like. Softwood pulp has shorter fiber length than hardwood pulp, and therefore is more suitable for use in terms of function, price, and so on. Providing the wrapping sheet 5 to surround the absorber 4 results in placing the wrapping sheet 5 between the inner surface sheet 3 and the absorber 4, and the wrapping sheet 5, which has excellent absorbability, allows the body fluids to diffuse quickly, and also prevent these body fluids from flowing back.

Furthermore, synthetic fiber may be mixed in the absorber 4. For this synthetic fiber, polyolefins such as polyethylene and polypropylene, polyesters such as polyethylene terephthalate and polybutylene terephthalate, polyamides such as nylon, and copolymers of these can be used. Also, two of these can be mixed and used. Furthermore, composite fiber of core-sheath fiber formed with fiber having a high melting point as the core and fiber having a low melting point as the sheath, side-by-side fiber, split fiber, and the like can also be used. For the synthetic fiber, for example, hydrophobic fiber subjected to surface treatment using a hydrophilic agent and thus given an affinity for body fluids is desirable for use.

As shown in the cross-sectional view of FIG. 3, the widthwise dimension of the inner surface sheet 3 is slightly longer than the width of the absorber 4, and only covers the absorber 4, and, on the outer side of that, side non-woven fabrics 7, which are different from the inner surface sheet 3, are provided. To be more specific, side non-woven fabrics 7 that are made of a non-woven fabric material subjected to water repellent or hydrophilic treatment as appropriate depending on the purpose of use such as preventing menstrual blood or vaginal discharge from permeating, improving the feeling of touch, and so forth, are provided. For the material of the side non-woven fabrics 7, materials that are formed by appropriate processing methods using natural fiber, synthetic fiber, recycled fiber or the like can be used. To eliminate the feeling of stiffness and prevent stuffiness, it is preferable to use a non-woven fabric that has a reduced basis weight and that is thus breathable preferable for use. To be more specific, it is desirable to use a non-woven fabric produced with a basis weight of 13 g/m² to 23 g/m². Furthermore, to make sure that the permeation of body fluids is prevented, a water-repellent-treated non-woven fabric that is coated with a silicon-based, paraffin-based, alkylchromic chloride-based water repellent, or the like is suitable for use.

As illustrated in FIG. 3, in the side non-woven fabrics 7, the outer portions of a widthwise middle portion are bonded from a predetermined inner position to the outer edges of the outer surface sheet 2, by an adhesive means such as hot melting, and, in both side portions of the absorber 4, the layered sheet portions of these side non-woven fabrics 7 and the outer surface sheet 2 form flap portions in which there is no intervening absorber 4. In these flap portions, a pair of left and right wing-like flaps W and W are formed in positions of the absorber's side portions that substantially correspond to the body fluid discharging part H of the person wearing the sanitary napkin 1 (see FIG. 2).

As illustrated in FIG. 2 and FIG. 3, on the non-skin-contacting surface (the outer surface of the outer surface sheet 2) of the main body portion where the absorber 4 is interposed between the inner surface sheet 3 and the outer surface sheet 2, multiple main body adhesive layers 8, or three main body adhesive layers 8 in the illustrations of the drawings, are formed by an appropriate coating pattern for fixing the absorbent article to the underwear. Furthermore, in each of the wing-like flaps W and W, a wing adhesive layer 9 is provided in the underwear-contacting surface (the outer surface of the outer surface sheet 2) so that, when attaching the sanitary napkin 1 to the underwear, the wing-like flaps W and W are folded back to the outer surface side (the outer surface sheet 2 side) at base end positions, wrapped around the crotch portion of the underwear, and thus fixed.

As for the adhesive to form the above adhesive layers 8 and 9, for example, one containing any of a styrene-based polymer, a tackifier, and a plasticizer as a main ingredient is suitable for use. Examples of the styrene-based polymer include a styrene-ethylene-butylene-styrene block copolymer, a styrene-butylene-styrene block copolymer, and a styrene-isobutylene-styrene copolymer, but only one type among these polymers may be used, or two or more types of these polymers may be blended. Among these, the styrene-ethylene-butylene-styrene block copolymer is preferable because it has excellent thermal stability. Furthermore, for the tackifier and the plasticizer, ones that are solid at room temperature are suitable for use. Examples of the tackifier include a C5 petroleum resin, a C9 petroleum resin, a dicyclopentadiene petroleum resin, a rosin petroleum resin, a polyterpene resin, a terpene phenol resin, and so on. Examples of the plasticizer include polymer plasticizers such as vinyl polymers and polyesters, in addition to monomer plasticizers such as tricresyl phosphate, dibutyl phthalate, dioctyl phthalate, and so on.

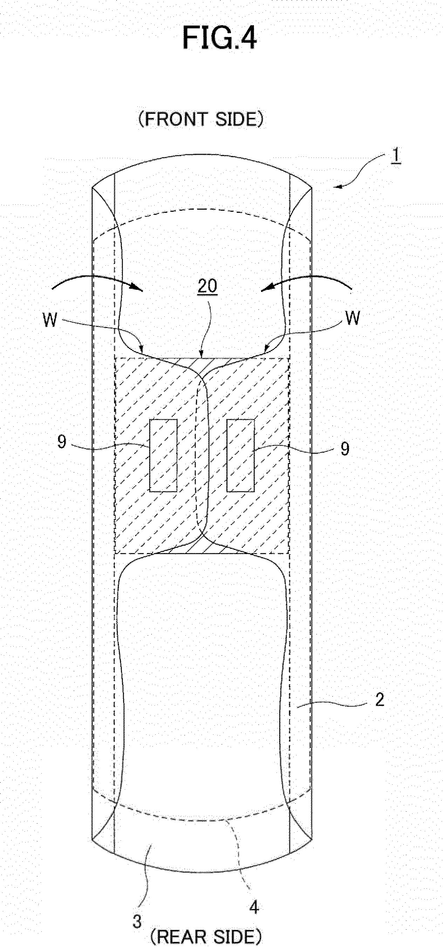

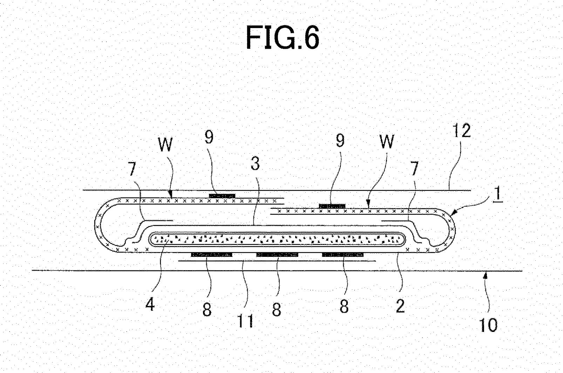

With this individually-wrapped sanitary napkin N, as illustrated in FIG. 4 to FIG. 6, the main body adhesive layers 8 are collectively covered by a main body separating sheet 11 as one piece, in a separable manner. The wing adhesive layers 9 and 9, provided in portions where each wing-like flap W is folded to the skin-contacting surface side, are covered by a wing separating sheet 12 that collectively covers these as one piece, in an inseparable manner. The main body separating sheet 11 is inseparably fixed to the wrapping sheet 10 layered on the outer surface thereof, and it is preferable if, when the sanitary napkin 1 is taken out of the individually-wrapped sanitary napkin N, the main body separating sheet 11 peels off from the main body adhesive layers 8 and left in the wrapping sheet 10. On the other hand, the wing separating sheet 12 may be separably fixed to the outer surface of the wrapping sheet 10 folded lengthwise, or may not be joined with the wrapping sheet 10.

As for the separating sheets 11 and 12, a paper or plastic sheet, in which a mold release liquid such as a silicone resin, a fluororesin, or a tetrafluoroethylene resin is applied or spray-applied, to the contacting surface with the slip-preventing adhesive layers 8 and 9, and the mold release process is performed thereon, can be used. In particular, it is preferable to use a plastic sheet as the wing separating sheet 12 because the dissipation of a functional chemical 20, which will be described later in detail, can be further reduced. Note that a film per se or a non-woven fabric per se may be used as long as it does not substantially reduce the adhesive strength, without applying any special mold release process.

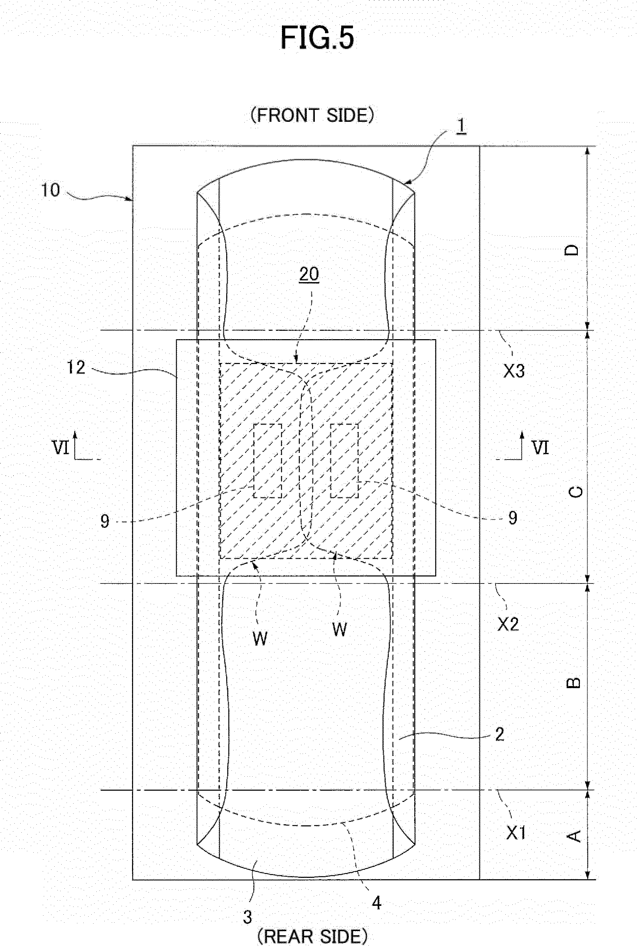

For the wrapping sheet 10, as illustrated in FIG. 5, a sheet-like member having a rectangular shape is used. To be more specific, a polyolefin film such as polypropylene and polyethylene, a film such as polyester and polyvinyl alcohol, a non-woven fabric, paper, and a polyethylene-laminated non-woven fabric may be used. If printing design is important, it is preferable to use a film. If texture, softness and the like are important, it is preferable to use a non-woven fabric or paper.

As illustrated in FIG. 5, the longitudinal dimension of the wrapping sheet 10 is formed so that at least the front edge of the wrapping sheet 10 extends beyond and ahead of the front end of the sanitary napkin 1, and the lateral dimension is formed slightly longer than the widthwise dimension in the state in which the left and right flap portions of the sanitary napkin 1 are folded to the inner surface sheet 3 side.

To wrap the sanitary napkin 1 individually with the wrapping sheet 10, first, as illustrated in FIG. 4, the left and right flap portions are folded to the inner surface sheet 3 side, the main body adhesive layers 8, provided on the outer surface sheet 2 side of the main body portion, are covered with the main body separating sheet 11, and then the wing adhesive layers 9 and 9, provided in the folded wing-like flaps W, are covered with the wing separating sheet 12. In the actual manufacturing process, the main body adhesive layers 8 are applied to the surface of the main body separating sheet 11 where the sanitary napkin is located, wing adhesive layers 9 and 9 are applied to the surface of the wing separating sheets 12 where the sanitary napkin is located, and then the sanitary napkin 1 is provided in these separating sheets 11 and 12, thereby allowing the adhesive to transfer to a predetermined surface of the sanitary napkin 1.

Following that, as illustrated in FIG. 5, in the portion of the sanitary napkin 1 located behind the wing-like flaps W, the rear end portion A is folded back with the wrapping sheet 10, at a widthwise fold line X1, to the sanitary napkin 1 side in the rear portion B near the wing-like flaps W. In this state in which the portion indicated as A and the portion indicated as B are piled on top of each other, these portions A and B are folded back, at a widthwise fold line X2, to the sanitary napkin 1 side in a portion C including the wing-like flaps W, and a portion D, located ahead of the wing-like flaps W, is folded back over the portion of the sanitary napkin 1 where the portion indicated as A and the portion indicated as B are piled on top of each other and where C is indicated, and folded in four, at a widthwise fold line X3. Then, as illustrated in FIG. 1, side edge sealing portions 13 and 13 are formed in a pair of open side edge portions of the wrapping sheet 10 by using an appropriate sealing means such as embossing pressure bonding, heat welding, or an adhesive, alone or in combination, and, if necessary, the widthwise central portion of the lengthwise edge of the wrapping sheet 10 is sealed by an adhesive fastening tape 14. By this means, an individually-wrapped sanitary napkin N is obtained.

### [Functional chemical]

In this individually-wrapped sanitary napkin N, as illustrated in FIG. 5, a functional chemical 20 having a specific function is contained in the inner surface sheet 3, in the area where the wing separating sheet 12, covering the wing adhesive layers 9 and 9 integrally, is layered. The wing adhesive layers 9 and 9 are provided in the left and right wing-like flaps W and W, folded to the inner surface sheet 3 side. In other words, the wing separating sheet 12 is layered on the skin-contacting surface side of the area where the functional chemical 20 is contained, and the wing separating sheet 12 covers the entirety of this area. It is preferable if the wing separating sheet 12 is directly layered on the skin-contacting surface side of the inner surface sheet 3 without anything intervening between the inner surface sheet 3 where the functional chemical 20 is contained and the wing separating sheet 12. However, it is also possible to interpose the wing-like flaps W and W that are folded on the inner surface sheet 3 side so as to wrap the sanitary napkin 1 individually.

The functional chemical 20 is a material that imparts a specific function such as a skin care function having moisturizing and humectant benefits on the skin, a deodorant or deodorant function, a fragrance function, a cooling or warming function, and so forth, to the person who wears the sanitary napkin 1. For example, a skin care agent, a deodorant, a fragrance agent, a cooling sensation agent, or the like can be used.

The functional chemical 20 may be in any form such as a liquid, a solid (a powder or the like), or a gel. When the functional chemical 20 assumes the form of a liquid or a gel, it can be applied as is. When the functional chemical 20 assumes the form of a solid such as a powder, this solid (powder) can be contained in the inner surface sheet 3 by a method of fixing it by using an adhesive or the like. The surface to contain the functional chemical 20 may be either the skin-contacting surface or the non-skin-contacting surface of the inner surface sheet 3, or the functional material 20 may be contained in both surfaces.

With the present individually-wrapped sanitary napkin N, the wing separating sheet 12 is layered over the entire surface of the skin-contacting surface side of the area where the functional chemical 20 is contained. Even in a state where the sanitary napkin 1 is folded small with the wrapping sheet 10 and wrapped individually, it is still possible to prevent the functional chemical 20 from transferring to an unintended portion of the sanitary napkin 1. Therefore, there is no risk that the functional chemical 20 is transferred to an unintended portion of the sanitary napkin 1, adheres to the clothes when wearing the sanitary napkin 1, and stains the clothes.

Furthermore, since the surface of the wing separating sheet 12 that contacts the wing adhesive layers 9 and 9 is subjected to a mold release process, the airtightness of the sheet itself is improved, and a water repellent effect is imparted. Furthermore, since this wing separating sheet 12 is layered over the entire surface of the area where the functional chemical 20 is contained, it is possible to securely reduce the diffusion of the fragrance ingredient of the fragrance, the volatilization of the skin care agent, and so forth. Therefore, the functional chemical 20 can be securely prevented from dissipating prior to use, and the effect of the functional chemical can be exerted optimally when in use.

The functional chemical 20 is preferably contained in an area including the area facing the body fluid discharging part H of the person wearing the sanitary napkin 1. That is, the functional chemical 20 is preferably provided in an area including the napkin's lengthwise range where the wing-like flaps W and W are provided in both side portions. By this means, a skin care agent, a deodorant, a cooling sensation agent, and so forth can be allowed to act on the body fluid discharging part H of the person wearing the sanitary napkin 1. Furthermore, when the sanitary napkin 1 is designed such that a fragrance or the like volatilizes to make a fragrance effect act on the person wearing the sanitary napkin 1, the action of removing the wing separating sheet 12 when opening the individually-wrapped sanitary napkin N allows the fragrance effect to be exerted more readily.

In the event side non-woven fabrics 7 and 7 are provided lengthwise in both side portions of the skin-contacting surface side of the sanitary napkin 1, as is the case with the present embodiment, the functional chemical 20 is preferably contained in the inner surface sheet 3 between the side non-woven fabrics 7 of both side portions. By this means, the functional chemical 20 acts directly on the skin surface of the person wearing the sanitary napkin 1, so that the effect of the functional chemical can be exerted more optimally.

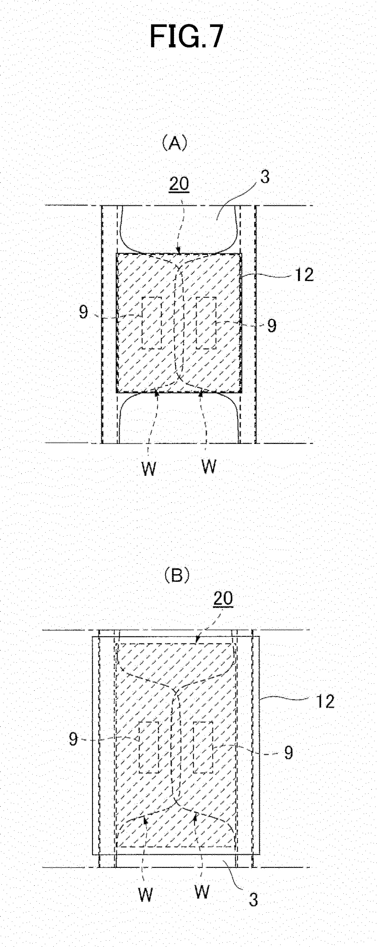

The wing separating sheet 12 can be formed in predetermined dimensions in accordance with the size of the functional chemical 20 as long as the wing separating sheet 12 has a size that at least integrally covers the wing adhesive layers 9 and 9 together, which are provided on the left and right wing-like flaps W and W. That is, as illustrated in FIG. 5, when the functional chemical 20 is formed over a longer area than the wing adhesive layers 9 and 9 in the napkin's lengthwise front and rear, the wing separating sheet 12 is formed to have substantially the same dimensions as or slightly larger dimensions than the functional chemical 20. As described above, the wing separating sheet 12 may be formed in a slightly larger size than the functional chemical 20 as illustrated in FIG. 5, or may be formed in substantially the same size as the functional chemical 20 as illustrated in FIG. 7(A). Also, as illustrated in FIG. 5, the functional chemical 20 may be provided between fold lines X2 and X3, which are provided for individual wrapping in the front and rear of the area where the wing-like flaps W and W are provided, or the functional chemical 20 may be extended and provided outside one or both of fold lines X2 and X3 as illustrated in FIG. 7 (B) .

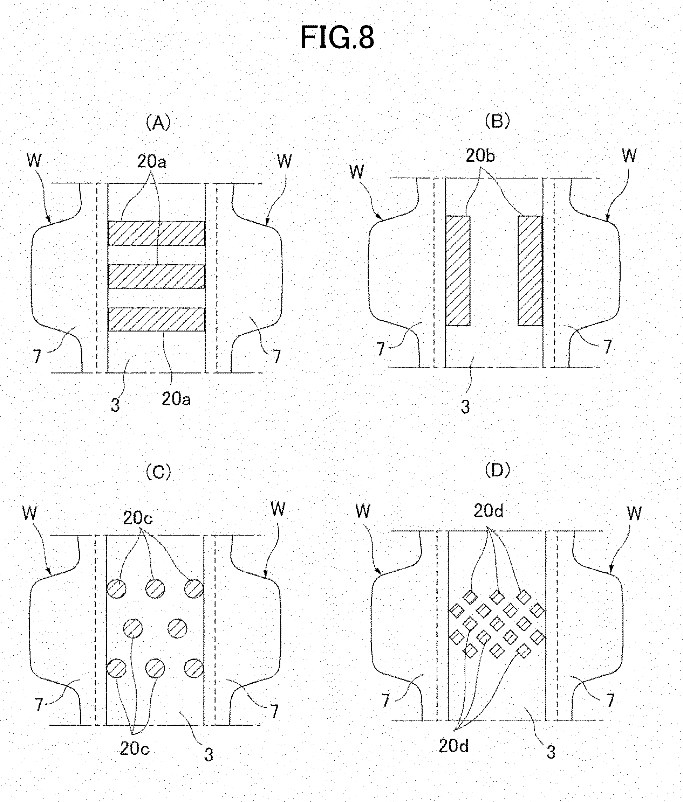

The functional chemical 20 may be provided over the entire surface of a predetermined area as illustrated in FIG. 5. Furthermore, multiple functional chemicals 20 may be provided at intervals in the longitudinal direction of the sanitary napkin 1 as illustrated in FIG. 8(A), or multiple functional chemicals 20 may be provided at intervals in the width direction, as illustrated in FIG. 8(B). In the embodiment illustrated in FIG. 8(A), three rectangular functional chemical-containing areas 20a that are long in the width direction of the sanitary napkin 1 are provided at intervals in the longitudinal direction. In the embodiment illustrated in FIG. 8B, two rectangular functional chemical-containing areas 20b that are long in the longitudinal direction of the sanitary napkin 1 are provided at intervals in the width direction.

Furthermore, as illustrated in FIG. 8(C) and FIG. 8(D), multiple functional chemicals 20 may be provided at intervals in the longitudinal direction and the width direction of the sanitary napkin 1. In the embodiment illustrated in FIG. 8(C), multiple circular functional chemical-containing areas 20c are provided in a houndstooth check pattern, and, in the embodiment illustrated in FIG. 8(D), multiple diamond-shaped functional chemical-containing areas 20d are provided in a houndstooth check pattern.

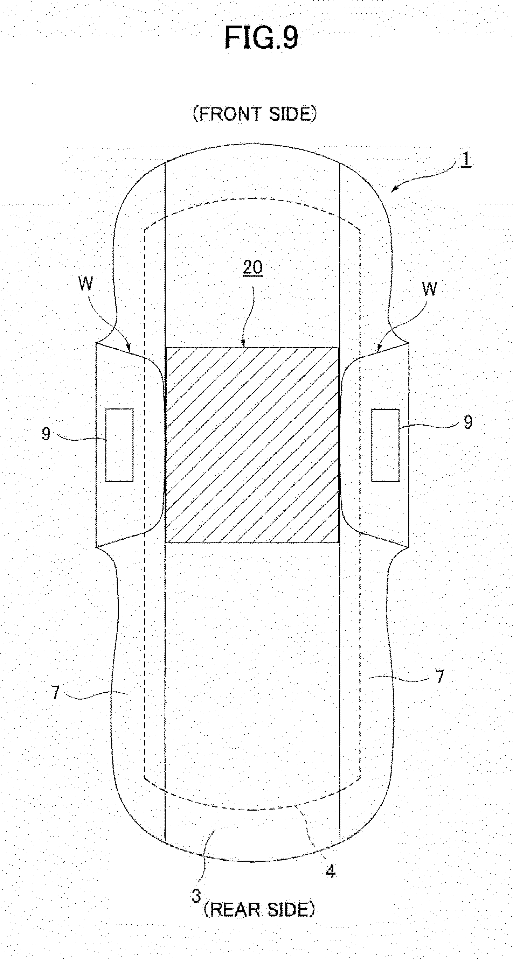

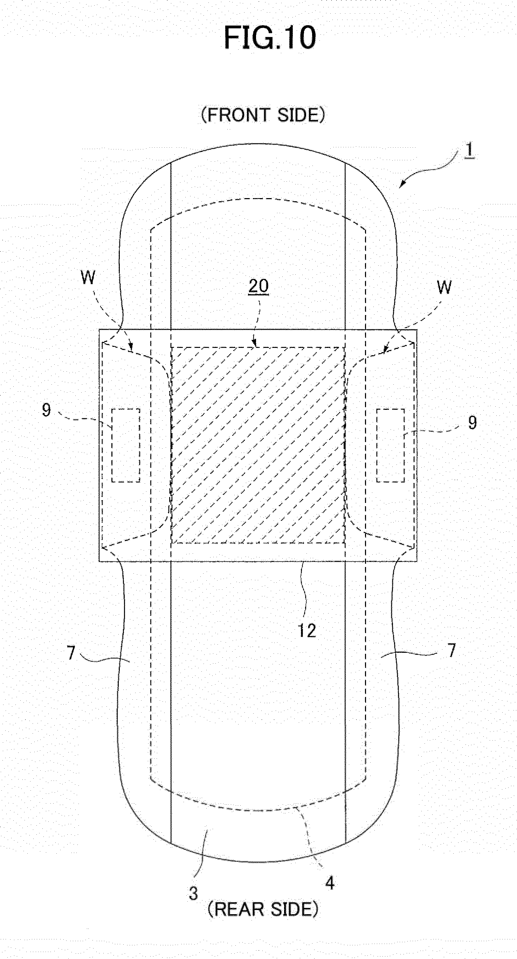

In the embodiment illustrated in FIG. 5, the wing-like flaps W and W are folded and directly layered on the skin-contacting surface side of the inner surface sheet 3 where the functional chemical 20 is contained, and thus there is a risk that the functional chemical 20 might transfer to these folded wing-like flaps W and W and adhere to the clothes when wearing the sanitary napkin 1. So, as illustrated in FIG. 9, the wing-like flaps W and W may be folded in both side portions not in direct contact with the inner surface sheet 3. That is, the wing-like flaps W and W may be folded such that each wing-like flap W is folded within the width of the side non-woven fabric 7, and the tip of each folded wing-like flap W is located in a position that substantially matches with the center-side edge of the side non-woven fabric 7 in the napkin's width direction or located in a position further outside than that. At this time, each of the wing adhesive layers 9 is provided in the folded portion of the corresponding wing-like flap W, so that, as illustrated in FIG. 9, the wing adhesive layers 9 and 9 are provided on the skin-contacting surface side of the sanitary napkin 1 when the wing-like flaps W and W are in a folded state. In this state, as illustrated in FIG. 10, the left and right wing adhesive layers 9 and 9 are integrally covered by the wing separating sheet 12.

In this way, by folding the wing-like flaps W and W in both side portions not in direct contact with the inner surface sheet 3, it becomes possible to prevent the functional chemical 20 from transferring to the wing-like flaps W and W, and thus prevent the functional chemical 20 from adhering to and soiling the clothes when wearing the sanitary napkin 1. Furthermore, since the wing separating sheet 12 is directly layered on the skin-contacting surface side of the inner surface sheet 3 where the functional chemical 20 is contained, the airtightness and water repellency of the wing separating sheet 12 make it possible to more securely prevent the functional chemical 20 from dissipating prior to use.

Here, according to the embodiment illustrated in FIG. 9, the sanitary napkin 1 is wrapped individually such that the wing-like flaps W and W are folded in portions that are different from the fold lines for folding the wing-like flaps W and W outwards so as to wrap it around the crotch portion of the underwear when wearing the sanitary napkin 1. Consequently, when creases are likely to be made while the sanitary napkin 1 is wrapped individually and make it difficult to fold the wing-like flaps W and W in a way to fit the crotch portion of the underwear when wearing the sanitary napkin 1, it is then preferable to provide the following means to assist the folding positions of the wing-like flaps W and W.

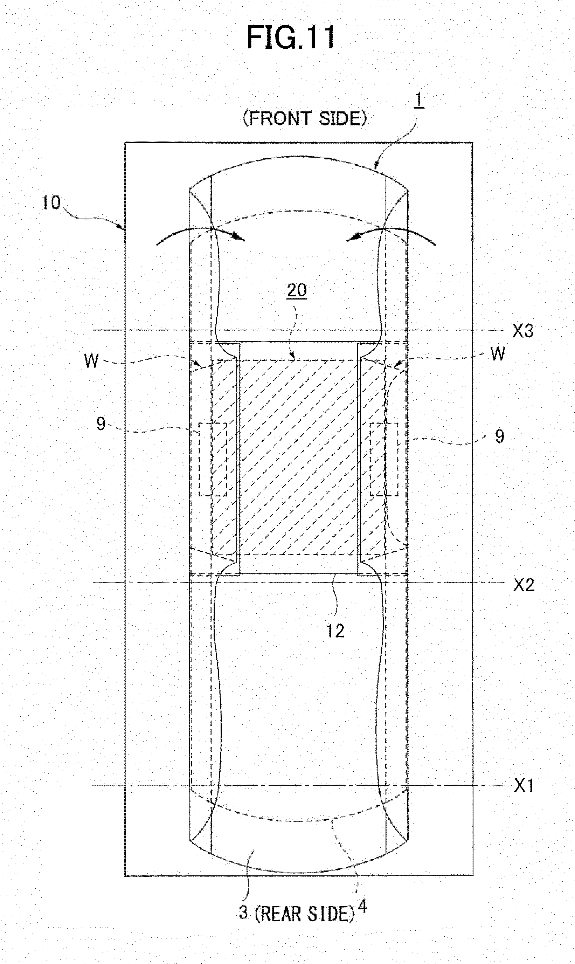

As a first means for assisting the folding positions of the wing-like flaps W and W when wearing the sanitary napkin 1, as illustrated in FIG. 11, the wing-like flaps W and W are folded with the wing separating sheet 12, along the fold lines where the wing-like flaps W and W are folded in a way to fit the crotch portion of the underwear when wearing the sanitary napkin 1. By this means, creases are gained along the fold lines the wing-like flaps W and W are folded back when wearing the sanitary napkin 1, and this make it easier to fold back the wing-like flaps W and W in a way to fit the crotch portion of the underwear when wearing the sanitary napkin 1. The folding direction may be the skin-contacting surface side of the sanitary napkin 1 as shown in the illustrated example, or may be the non-skin-contacting surface side of the sanitary napkin 1, which is the same as the folding direction of the wing-like flaps W and W when wearing the sanitary napkin 1.

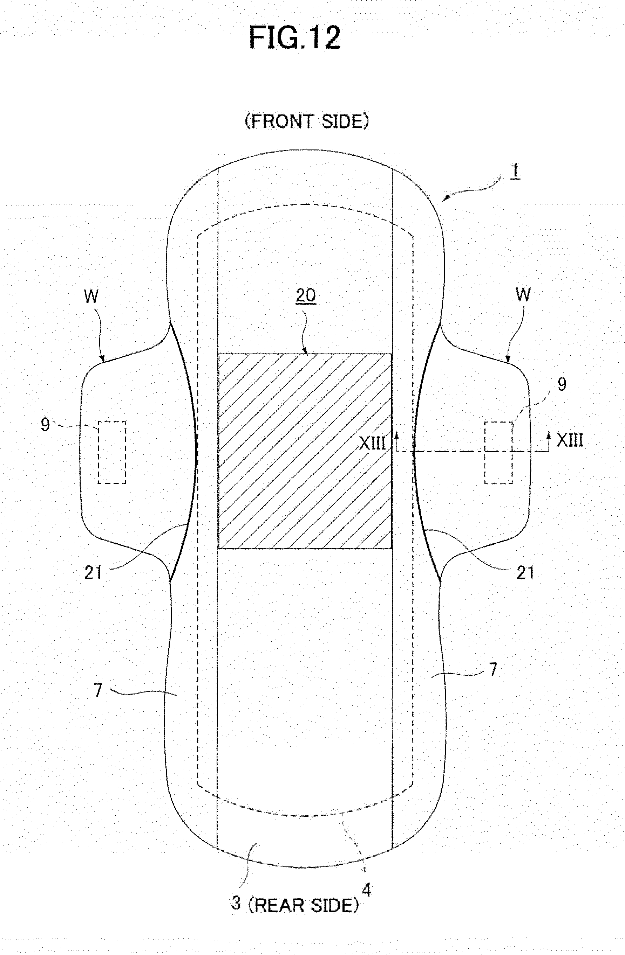

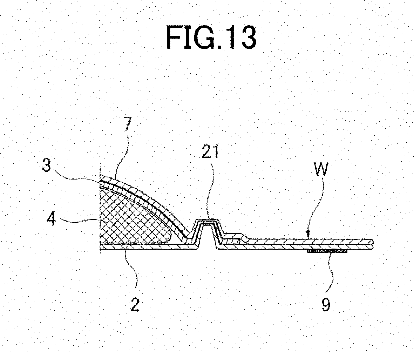

Furthermore, as a second means for assisting the folding position of the wing-like flaps W and W, as illustrated in FIG. 12 and FIG. 13, fold assist lines 21 are provided along the fold lines for folding back the wing-like flaps W and W in a way to fit the crotch portion of the underwear when wearing the sanitary napkin 1. The fold assist lines 21 are created by using heat sealing or by using embossed portions, and, in the illustrated examples, the fold assist lines 21 are formed by using embossed portions recessed from the outer surface sheet 2 side to the side non-woven fabric 7 side. Note that these embossed portions may be formed, in cross-sectional view, to be recessed from the side non-woven fabric 7 side to the outer surface sheet 2 side. The planar shape of the fold assist line 21 is preferably shaped to pass through the front and rear base end portions of each wing-like flap W so as to fit the outer line of the crotch portion of the underwear, and form an arc line that is curved inward in the width direction, so as to be in contact with the side edge of the absorber 4 in the center portion of the wing-like flap W in the longitudinal direction of the napkin. By providing the fold assist lines 21, the wing-like flaps W and W can be easily folded back with the fold assist lines 21 as base points.

### [Other embodiments]

Although a four-fold individually-wrapped sanitary napkin N, in which the area behind the wing-like flaps W is folded twice at fold lines X1 and X2, and the area in the front of the wing-like flaps W is folded once at fold line X3 has been illustrated with the above embodiments, the present invention might as well provide a tri-fold individually-wrapped sanitary napkin N, in which the area behind the wing-like flaps W is folded once and the area in the front of the wing-like flaps W is folded once, or provide an individually-wrapped sanitary napkin N that is five-fold or more.

### DESCRIPTION OF THE REFERENCE NUMERALS

- 1: Sanitary napkin,
- 2: Outer surface sheet,
- 3: Inner surface sheet,
- 4: Absorber,
- 5: Wrapping sheet,
- 7: Side non-woven fabric,
- 8: Body adhesive layer,
- 9: Wing adhesive layer,
- 10: Wrapping sheet,
- 11: Body separating sheet,
- 12: Wing separating sheet,
- 13: Side edge sealing portion,
- 14: Fastening tape,
- 20: Functional chemical,
- 21: Fold assist line,
- N: Individually-wrapped sanitary napkin,
- W: Wing-like flap W

## Claims

1. An individually-wrapped absorbent article, in which an absorbent article is folded lengthwise with a wrapping sheet and wrapped individually, the absorbent article comprising an inner surface sheet that constitutes a skin-contacting surface layer, and a pair of wing-like flaps that have wing adhesive layers for fixing the wing-like flaps to underwear, wherein the wing-like flaps are both folded to a skin-contacting surface side, and the wing adhesive layers, provided in folded portions of the wing-like flaps, are collectively covered by a separating sheet as one piece, and
wherein a functional chemical having a specific function is contained in the inner surface sheet in an area where the separating sheet is layered.

2. The individually-wrapped absorbent article according to claim 1, wherein the functional chemical is provided in at least one of the following manners:
the functional chemical is provided over an entire surface of a predetermined area;
a plurality of functional chemicals are provided at intervals in the longitudinal direction or a width direction of the absorbent article; and
the plurality of functional chemicals are provided at intervals in the longitudinal direction and the width direction of the absorbent article.

3. The individually-wrapped absorbent article according to one of claim 1 and claim 2, wherein the wing-like flaps are folded in both side portions that do not directly contact the inner surface sheet.

4. The individually-wrapped absorbent article according to claim 3, wherein the wing-like flaps are folded with the separating sheet at fold line positions where the wing-like flaps are folded back along a crotch portion of the underwear when the absorbent article is worn.

5. The individually-wrapped absorbent article according to claim 3, wherein fold assist lines are provided at the fold line positions where the wing-like flaps are folded back along the crotch portion of the underwear when the absorbent article is worn.

6. The individually-wrapped absorbent article according to any one of claims 1 to 5,
wherein side non-woven fabrics are provided lengthwise in both side portions of the skin-contacting surface side, and
wherein the functional chemical is contained in the inner surface sheet between the side non-woven fabrics of the both side portions.
